(19) **Europäisches Patentamt European Patent Office Office européen des brevets**

(11) **EP 2 871 246 A1**

(12) **EUROPEAN PATENT APPLICATION**
published in accordance with Art. 153(4) EPC

(43) Date of publication:
**13.05.2015 Bulletin 2015/20**

(21) Application number: **13812846.7**

(22) Date of filing: **03.07.2013**

(51) Int Cl.:
*C13K 1/02* (2006.01)     *B01J 21/18* (2006.01)
*B09B 3/00* (2006.01)

(86) International application number:
**PCT/JP2013/068277**

(87) International publication number:
**WO 2014/007295 (09.01.2014 Gazette 2014/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **03.07.2012 JP 2012149152**
**30.11.2012 JP 2012262096**

(71) Applicants:
• **Showa Denko K.K.**
**Tokyo 105-8518 (JP)**
• **National University Corporation Hokkaido University**
**Sapporo-shi, Hokkaido 060-0808 (JP)**

(72) Inventors:
• **FUJITA Ichiro**
**Tokyo 105-8518 (JP)**
• **FUKUOKA Atsushi**
**Sapporo-shi**
**Hokkaido 060-0808 (JP)**
• **KOBAYASHI Hirokazu**
**Sapporo-shi**
**Hokkaido 060-0808 (JP)**
• **YABUSITA Mizuho**
**Sapporo-shi**
**Hokkaido 060-0808 (JP)**

(74) Representative: **Strehl Schübel-Hopf & Partner**
**Maximilianstrasse 54**
**80538 München (DE)**

(54) **METHOD FOR DECOMPOSING PLANT BIOMASS, AND METHOD FOR PRODUCING GLUCOSE**

(57)     The present invention provides a method of hydrolyzing a plant biomass having high yield of glucose and high selectivity of glucose, which method comprises a step of heating a mixture containing a plant biomass, a solid catalyst for catalyzing hydrolysis of the biomass, an inorganic acid, and water. Hydrochloric acid can be preferably used as the inorganic acid. The pH of the mixture is adjusted preferably to 1.0 to 4.0, more preferably 2.0 to 3.0. Activated carbon and the like can be used as the solid catalyst.

[FIG. 1]

**Description**

Technical Field

The present invention relates to a method of hydrolyzing a plant biomass. More particularly, the present invention relates to a method of hydrolyzing a polysaccharide derived from a plant biomass through a reaction using a solid catalyst at a high saccharification yield, and to a method of producing glucose.

Background Art

In recent years, many studies have been made on use of useful substances converted from recyclable biomass resources produced from plants and the like. Cellulose contained in a plant biomass as a main component is characterized by being insoluble in water or a usual solvent and being persistent because it is a polymer formed of β-1,4-linked glucose units, forms hydrogen bonds within and between molecules, and thus exhibits high crystallinity. In recent years, a study on a reaction using a solid catalyst that is recyclable and can reduce an environmental burden has been made as a cellulose hydrolysis method instead of a sulfuric acid method or an enzyme method.

The hydrolysis reaction of cellulose through a hydrothermal reaction using a solid catalyst is a solid-solid reaction, and a rate of the reaction is limited by contact property of the catalyst and cellulose (substrate). Therefore, in order to realize a highly-efficient reaction, studies have been made on, for example, a treatment method of improving reactivity and a highly active catalyst.

For example, as a method of improving reactivity in a solid-solid reaction system, there are given a method involving mixing and preheating a pulverized substrate, a catalyst, and preheating steam (JP 2008-297229 A; Patent Document 1) and a method involving allowing a catalyst and a substrate to react under irradiation with microwaves (JP 2010-98994 A; Patent Document 2).

However, Patent Document 1 discloses that about 70% of cellulose is degraded, but does not specifically describe the yield of a sugar obtained as a degraded product, and the effect is unknown. In addition, in Patent Document 2, the yield of glucose is about 30%, and a high reaction yield has not been achieved. Further, it is necessary to introduce an expensive microwave irradiation apparatus, and the method is problematic in practicality.

As a method of improving saccharification performance through modification of a solid catalyst, there is given a method involving using as the solid catalyst an activated carbon solid acid catalyst subjected to sulfuric acid treatment (JP 2009-201405 A; Patent Document 3). Although the method of Patent Document 3 exhibits an effect of the catalyst subjected to sulfuric acid treatment, the yield of glucose is still about 40%. For practical use, the yield of glucose needs to be further improved.

As a method of improving reactivity in a pseudo-liquid-solid reaction system, there is given a method involving adding cellulose to a cluster acid catalyst in a pseudo-molten state to perform hydrolysis (JP 2008-271787 (US 8,382,905 B2); Patent Document 4). However, the method of Patent Document 4 is problematic in practicality because of difficult control of a water content during a reaction, necessity of many steps for separation of the catalyst from the product, and use of an organic solvent.

As a method of improving a saccharification yield in a hydrolysis reaction of cellulose through hydrothermal treatment without using a solid catalyst, there is given a method involving putting a raw material containing cellulose and an aqueous solution containing an inorganic acid into contact with each other, followed by heating and pressure treatment, to achieve a yield of glucose of 60% or more (JP 2011-206044 A; Patent Document 5). However, the method of Patent Document 5 uses as the inorganic acid perchloric acid at a high concentration of 0.1 mol/L, and thus a reaction liquid has an extremely low pH of from 0.8 to 0.9. Therefore, the method of Patent Document 5 is problematic in practicality because of cost of the acid to be added, necessity of neutralization and purification treatment after a reaction, corrosion of a device material, and the like.

For the above-mentioned reasons, it has been desired to establish a method of saccharifying cellulose that can achieve a high yield of glucose without an excessive burden of separation and purification after saccharification, in a hydrolysis reaction of a plant biomass through a hydrothermal reaction using a solid catalyst.

Prior Art Documents

Patent Documents

[Patent Document 1] JP 2008-297229 A
[Patent Document 2] JP 2010-98994 A

[Patent Document 3] JP 2009-201405 A
[Patent Document 4] JP 2008-271787 A (US 8,382,905 B2)
[Patent Document 5] JP 2011-206044 A

Summary of Invention

Problem to be Solved by the Invention

It is an object of the present invention to provide a method of hydrolyzing a plant biomass that can improve a yield of glucose and selectivity of glucose.

Means to Solve the Problem

As a result of diligent study aimed at solving the problems described above, the inventors of the present invention have found that, in a hydrolysis reaction of a plant biomass using a solid catalyst, the yield of glucose and selectivity of glucose can be improved by conducting the reaction under the presence of an inorganic acid. Thus, the present invention has been completed.

That is, the present invention includes a method of hydrolyzing a plant biomass according to the following items [1] to [10] and a method of producing glucose according to the following item [11] .

[1] A method of hydrolyzing a plant biomass, including a step of heating a mixture containing a plant biomass, a solid catalyst for catalyzing hydrolysis of the biomass, an inorganic acid, and water.

[2] The method of hydrolyzing a plant biomass according to [1] above, in which the mixture containing a plant biomass, a solid catalyst, an inorganic acid, and water has a pH of from 1.0 to 4.0.

[3] The method of hydrolyzing a plant biomass according to [2] above, in which the mixture containing a plant biomass, a solid catalyst, an inorganic acid, and water has a pH of from 2.0 to 3.0.

[4] The method of hydrolyzing a plant biomass according to any one of [1] to [3] above, in which the inorganic acid includes at least one kind selected from hydrochloric acid, sulfuric acid, nitric acid, phosphoric acid, and boric acid.

[5] The method of hydrolyzing a plant biomass according to any one of [1] to [3] above, in which the inorganic acid is hydrochloric acid.

[6] The method of hydrolyzing a plant biomass according to any one of [1] to [5] above, in which the heating is performed at a maximum heating (reaction) temperature of from 170°C to 200°C and a retention time at the temperature is from 0 to 120 minutes.

[7] The method of hydrolyzing a plant biomass according to any one of [1] to [6] above, in which the heating is performed so that, in a graph with a vertical axis representing a reaction temperature and a horizontal axis representing time, a product of temperature and time for a portion above 160°C ((treatment temperature-160°C)×time) is from 200 to 800°C·min.

[8] The method of hydrolyzing a plant biomass according to any one of [1] to [7] above, in which the solid catalyst includes a carbon material.

[9] The method of hydrolyzing a plant biomass according to [8] above, in which the carbon material includes alkali-activated carbon, steam-activated carbon, or mesoporous carbon.

[10] The method of hydrolyzing a plant biomass according to any one of [1] to [9] above, in which the plant biomass includes cellulose.

[11] A method of producing glucose, comprising performing the method of hydrolyzing a plant biomass according to any one of [1] to [10] above.

Effects of the Invention

According to the method of hydrolyzing a plant biomass of the present invention, the yield of glucose and selectivity of glucose can be improved.

Brief Description of Drawings

FIG. 1 is a graph showing results of Examples 1 to 9 and Comparative Example 1.
FIG. 2 is a graph showing results of Comparative Examples 4 to 8.

Mode for Carrying out the Invention

The present invention is hereinafter described in detail.

[Plant biomass (solid substrate)]

In the present description, the "plant biomass" (hereinafter sometimes referred to as solid substrate) is, for example, a biomass such as rice straw, straw, sugarcane straw, chaff, bagasse, a broadleaf tree, bamboo, a coniferous tree, kenaf, furniture waste, construction waste, waste paper, or a food residue, which mainly contains cellulose or hemicellulose. It should be noted that the term "biomass" generally refers to "recyclable organic resource of biologic origin, excluding fossil resources."

The plant biomass to be used may be a plant biomass subjected to purification treatment or a plant biomass not subjected to purification treatment. The plant biomass subjected to purification treatment is, for example, one that is obtained by subjecting the plant biomass to treatment such as alkali steam treatment, alkaline sulfite steam treatment, neutral sulfite steam treatment, alkaline sodium sulfide steam treatment, or ammonia steam treatment, and then to delignification treatment by solid-liquid separation and water washing, and that contains two or more polysaccharides out of cellulose, hemicellulose, and lignin. Further, the plant biomass may be industrially prepared cellulose, xylan, cellooligosaccharide, or xylooligosaccharide. The plant biomass may contain an ash content such as silicon, aluminum, calcium, magnesium, potassium, or sodium, which is derived from the plant biomass, as an impurity.

The plant biomass may be in a dry form or a wet form, and may be crystalline or non-crystalline. It is desired that the plant biomass be pulverized prior to a reaction. The pulverization increases contact property with a solid catalyst, and thereby promotes a hydrolysis reaction. Therefore, it is desired that the plant biomass have a shape and size appropriate for the pulverization. As such shape and size, there is given, for example, a powder shape having a particle diameter of 20 $\mu$m or more and several thousand micrometers or less.

[Solid catalyst]

The solid catalyst is not particularly limited as long as the catalyst can hydrolyze the plant biomass, but preferably has an activity to hydrolyze a glycoside bond typified by $\beta$-1, 4 glycosidic bonds between glucose units that form cellulose contained as a main component.

Examples of the solid catalyst include a carbon material and a transition metal. One kind of those solid catalysts may be used alone, or two or more kinds thereof may be used in combination.

Examples of the carbon material include activated carbon, carbon black, and graphite. One kind of those carbon materials may be used alone, or two or more kinds thereof may be used in combination. Regarding the shape of the carbon material, from the viewpoint of improving reactivity by increasing an area for contact with a substrate, the carbon material is preferably porous and/or particulate. From the viewpoint of promoting hydrolysis by expressing an acid center, the carbon material preferably has a functional group such as a phenolic hydroxyl group, a carboxyl group, a sulfo group, or a phosphate group in its surface. Examples of a porous carbon material having a functional group in its surface include a wood material such as coconut husk, bamboo, pine, walnut husk, or bagasse; and activated carbon prepared by a physical method involving treating coke or phenol at high temperature with a gas such as steam, carbon dioxide or air, or by a chemical method involving treating coke or phenol at high temperature with a chemical reagent such as an alkali or zinc chloride.

Examples of the transition metal include ruthenium, platinum, rhodium, palladium, iridium, nickel, cobalt, iron, copper, silver and gold. One kind of those transition metals may be used alone, or two or more kinds thereof may be used in combination. One selected from platinum group metals including ruthenium, platinum, rhodium, palladium, and iridium is preferred from the viewpoint of having a high catalytic activity, and one selected from ruthenium, platinum, palladium, and rhodium is particularly preferred from the viewpoints of having a high rate of conversion of cellulose and selectivity of glucose.

[Pulverization of plant biomass]

Cellulose, which is a main component of a plant biomass, exhibits crystallinity, because two or more cellulose molecules are bonded to each other through hydrogen bonding. In the present invention, such cellulose exhibiting crystallinity may be used as a raw material, but cellulose that is subjected to treatment for reducing crystallinity and thus has reduced crystallinity may be used. As the cellulose having reduced crystallinity, cellulose in which the crystallinity is partially reduced or cellulose in which the crystallinity is completely or almost completely lost may be used. The kind of the treatment for reducing crystallinity is not particularly limited, but treatment for reducing crystallinity capable of breaking the hydrogen bonding and at least partially generating a single-chain cellulose molecule is preferably employed.

By using as the raw material cellulose at least partially containing the single-chain cellulose molecule, hydrolysis efficiency can be significantly improved.

As a method of physically breaking the hydrogen bonding between cellulose molecules, there is given, for example, pulverization treatment. The pulverization means is not particularly limited as long as the means has a function to enable fine pulverization. For example, the mode of the apparatus may be a dry mode or a wet mode. In addition, the pulverization system of the apparatus maybe a batch system or a continuous system. Further, the pulverization force of the apparatus may be provided by any of impact, compression, shearing, friction, and the like.

Examples of the apparatus that may be used in the pulverization treatment include: tumbling ball mills such as a pot mill, a tube mill, and a conical mill; vibrating ball mills such as a circular vibration type vibration mill, a rotary vibration mill, and a centrifugal mill; mixing mills such as a media agitating mill, an annular mill, a circulation type mill, and a tower mill; jet mills such as a spiral flow jet mill, an impact type jet mill, a fluidized bed type jet mill, and a wet type jet mill; shear mills such as a Raikai mixer and an angmill; colloid mills such as a mortar and a stone mill; impact mills such as a hammer mill, a cage mill, a pin mill, a disintegrator, a screen mill, a turbo mill, and a centrifugal classification mill; and a planetary ball mill as a mill of a type that employs rotation and revolution movements.

Hydrolysis is a reaction between a solid substrate and a solid catalyst, and a rate of the reaction is limited by contact property between the substrate and the catalyst. Therefore, as a method of improving reactivity, preliminarily mixing the solid substrate and the solid catalyst and then performing simultaneous pulverization treatment is also effective.

The simultaneous pulverization treatment may include pre-treatment for reducing the crystallinity of the substrate in addition to the mixing. From such viewpoint, the pulverization apparatus to be used is preferably a tumbling ball mill, a vibrating ball mill, a mixing mill, or a planetary ball mill, which is used for the pre-treatment for reducing the crystallinity of the substrate, more preferably a pot mill classified as the tumbling ball mill, a media agitating mill classified as the mixing mill, or the planetary ball mill. Further, the reactivity tends to increase when a raw material obtained by the simultaneous pulverization treatment for the solid catalyst and the solid substrate has a high bulk density. Therefore, it is more preferred to use the tumbling ball mill, the mixing mill, or the planetary ball mill that can apply a strong compression force enough to allow a pulverized product of the solid catalyst to dig into a pulverized product of the solid substrate.

A ratio between the solid catalyst and the solid substrate to be subjected to the simultaneous pulverization treatment is not particularly limited, but from the viewpoints of hydrolysis efficiency in a reaction, a decrease in a substrate residue after the reaction, and a recovery rate of a produced sugar, is preferably 1 to 100 parts by mass, more preferably 1 to 10 parts by mass of the solid catalyst with respect to 100 parts by mass of the solid substrate.

In each of the raw material obtained by separately pulverizing the substrate and the raw material obtained by simultaneously pulverizing the substrate and the catalyst, the average particle diameter after the fine pulverization (median diameter: particle diameter (D50) at a point where the cumulative volume curve determined based on the total powder volume defined as 100% crosses 50%) is from 1 to 100 $\mu$m, preferably from 1 to 30 $\mu$m, more preferably from 1 to 20 $\mu$m from the viewpoint of improving reactivity.

For example, when the particle diameter of a raw material to be treated is large, in order to efficiently perform the fine pulverization, preliminary pulverization treatment maybe performed before the fine pulverization with, for example: a coarse crusher such as a shredder, a jaw crusher, a gyratory crusher, a cone crusher, a hammer crusher, a roll crusher or a roll mill; or a medium crusher such as a stamp mill, an edge runner, a cutting/shearing mill, a rod mill, an autogenous mill or a roller mill. The time for treating the raw material is not particularly limited as long as the raw material can be homogeneously and finely pulverized by the treatment.

[Inorganic acid]

An inorganic acid is preferably hydrochloric acid, sulfuric acid, nitric acid, phosphoric acid or boric acid, and those inorganic acids may be used in combination. By adding such inorganic acid, the saccharification yield and selectivity of glucose in the hydrolysis reaction of the plant biomass using the solid catalyst can be improved. Hydrochloric acid is particularly preferred because of having a high effect of improving the selectivity of glucose.

The inorganic acid may be added by using a pH after its addition as an indicator. After the addition of the inorganic acid, a mixture containing the plant biomass, the solid catalyst, and water preferably has a pH of from 1.0 to 4.0. From the viewpoint of achieving a high saccharification yield and suppressing formation of an excessive degradation product, the pH is more preferably from 2.0 to 4.0, most preferably from 2.0 to 3.0.

The pH was measured with a pH meter D-51 (manufactured by HORIBA, Ltd.) subjected to three-point calibration using pH standard 100-4, 100-7, and 100-9 manufactured by HORIBA, Ltd., by soaking a glass electrode of the instrument in a sample solution of 25°C filled in a glass bottle, and then, briefly stirring the solution, leaving the solution to stand still, and waiting until the solution becomes stable (for about 1 minute).

[Hydrolysis reaction]

The hydrolysis using as a substrate a polysaccharide derived from the plant biomass is performed by heating the substrate under the presence of the catalyst, the inorganic acid, and water preferably at a temperature that allows for a pressurized state. As the heating temperature that allows for a pressurized state, for example, a range of from 110 to 380°C is appropriate. In the case where the plant biomass is cellulose, a relatively high temperature is preferred from the viewpoint of promptly performing its hydrolysis and suppressing conversion of glucose, which is a product, into another sugar. In this case, for example, it is appropriate to set the maximum heating (reaction) temperature within a range of from 170 to 320 °C, more preferably from 170 to 200 °C, still more preferably from 170 to 190°C. In addition, a retention time at the temperature is preferably from 0 to 120 minutes.

In addition, it is preferred that, in a graph with a vertical axis representing a reaction temperature and a horizontal axis representing time, an area of a portion above 160°C (hereinafter referred to as "product of temperature and time above 160°C") be from 200 to 800°C·min. It should be noted that, while the product of temperature and time above 160°C may be determined through integration of a difference between a treatment temperature and 160°C (treatment temperature-160°C) with respect to time for a portion above 160°C, the product is represented by the following equation in the case where temperature increase and temperature decrease are linear, and a chevron or trapezoidal graph is provided.

```
Product of temperature and time above 160°C=

    {(maximum reaction temperature-160°C)/rate of temperature

increase (°C/min)×(maximum reaction temperature-160°C)/2}

    +{(maximum reaction temperature-160°C)×retention time at

maximum reaction temperature (min)}

    +{(maximum reaction temperature-160°C)/rate of temperature

decrease (°C/min)×(maximum reaction temperature-160°C)/2}
```

When cellulose is taken as an example of the plant biomass in the saccharification method of the present invention, its hydrolysis is usually carried out in a closed vessel such as an autoclave. Therefore, even if the pressure at the start of the reaction is ordinary pressure, the reaction system becomes a pressurized state when heated at the above-mentioned temperature. Further, the closed vessel may be pressurized before the reaction or during the reaction to perform the reaction. The pressure for pressurization is, for example, from 0.1 to 30 MPa, preferably from 1 to 20 MPa, more preferably from 2 to 10 MPa. In addition to the closed vessel, the reaction liquid may be heated and pressurized to perform the reaction while the reaction liquid is allowed to flow by a high-pressure pump.

The amount of water for hydrolysis is at least one necessary for hydrolysis of the total amount of cellulose. In consideration of, for example, fluidity and stirring property of the reaction mixture, the amount of water may be from 1 to 500 times, preferably from 2 to 200 times as large as the mass of cellulose.

The atmosphere of the hydrolysis is not particularly limited. From an industrial viewpoint, the hydrolysis is preferably carried out under an air atmosphere, or may be carried out under an atmosphere of gas other than air, such as oxygen, nitrogen, or hydrogen, or a mixture thereof.

From the viewpoint of increasing the yield of glucose, the heating for hydrolysis is preferably completed at the point when the rate of conversion of cellulose by hydrolysis falls within a range of from 10 to 100% and the selectivity of glucose falls within a range of from 20 to 90%. The point when the rate of conversion of cellulose by hydrolysis falls within a range of from 10 to 100% and the selectivity of glucose falls within a range of from 20 to 90% varies depending on the heating temperature, the type and amount of the catalyst to be used, the amount of water (ratio relative to cellulose), the type of cellulose, the stirring method and conditions, and the like. Therefore, the point may be determined based on an experiment after determination of the conditions. The heating time under usual conditions falls within, for example, a range of from 5 to 60 minutes, preferably from 5 to 30 minutes after the start of the heating for the hydrolysis reaction, but the time is not limited to the range. In addition, the heating for hydrolysis is suitably completed at the point when the rate of conversion of cellulose by hydrolysis falls within a range of preferably from 30 to 100%, more preferably from 40 to 100%, still more preferably from 50 to 100%, most preferably from 55 to 100% and the selectivity of glucose falls within a range of preferably from 25 to 90%, more preferably from 30 to 90%, most preferably from 40 to 90%.

The hydrolysis reaction may be carried out in a batch fashion or a continuous fashion. The reaction is preferably carried out while stirring the reaction mixture.

In the present invention, it is possible to produce a sugar-containing solution that contains glucose as a main component and has a reduced amount of an excessive degradation product such as 5-hydroxymethylfurfural by performing a hydrolysis reaction at a relatively high temperature for a relatively short time.

After completion of heating, the reaction liquid is preferably cooled from the viewpoint of suppressing conversion of glucose into another sugar to increase the yield of glucose. From the viewpoint of increasing the yield of glucose, the cooling of the reaction liquid is carried out under conditions where the selectivity of glucose is maintained in a range of preferably from 20 to 90%, more preferably from 25 to 90%, still more preferably from 30 to 90%, most preferably from 40 to 90%.

From the viewpoint of increasing the yield of glucose, the cooling of the reaction liquid is preferably carried out as fast as possible to a temperature at which conversion of glucose into another sugar is not substantially caused. For example, the cooling may be carried out at a rate in a range of from 1 to 200°C/min and is preferably carried out at a rate in a range of from 10 to 150°C/min. The temperature at which conversion of glucose into another sugar is not substantially caused is, for example, 150 °C or less, preferably 110°C or less. That is, the reaction liquid is suitably cooled to 150°C or less at a rate in a range of from 1 to 200°C/min, preferably from 10 to 150°C/min, more suitably cooled to 110°C or less at a rate in a range of from 1 to 200°C/min, preferably from 10 to 150°C/min.

Examples

The present invention is hereinafter described in more details by way of Examples and Comparative Examples. However, the present invention is by no means limited to the descriptions of Examples and Comparative Examples.

[Solid catalyst]

Coke was subjected to heating treatment at 700°C, followed by fine pulverization with a jet mill. Then, potassium hydroxide was added thereto, and the resultant was again subjected to heating treatment at 700°C to be activated. After washed with water, the obtained activated coke was neutralized with hydrochloric acid and further boiled in hot water. After that, the resultant was dried and sieved. Thus, an alkali-activated porous carbon material (median diameter: 13 $\mu$m) having a particle diameter of 1 $\mu$m or more and 30 $\mu$m or less was obtained. The obtained solid catalyst is hereinafter referred to as carbon catalyst.

[Mixed and pulverized raw material]

10.00 g of Avicel (microcrystalline cellulose manufactured by Merck Co.), serving as the solid substrate, and 1.54 g of the carbon catalyst (mass ratio between the substrate and the catalyst: 6.5:1.0) were loaded in a 3, 600 mL-volume ceramic pot mill together with 2,000 g of alumina balls each having a diameter of 1.5 cm. The ceramic pot mill was set to a desktop pot mill rotating table (manufactured by NITTO KAGAKU Co., Ltd., desktop pot mill type AN2-51S). The mixture was simultaneously mixed and pulverized through ball mill treatment at 60 rpm for 48 hours. The obtained raw material is hereinafter referred to as mixed and pulverized raw material.

[Separately pulverized raw material]

3. 00 g of Avicel (microcrystalline cellulose manufactured by Merck Co.), serving as the solid substrate, were loaded in a 500 mL-volume ceramic pot mill together with 300 g of zirconia balls each having a diameter of 1.5 cm. The ceramic pot mill was set to a desktop pot mill rotating table (manufactured by IRIE SHOKAI Co., Ltd., desktop pot mill type V-1M) . The content was pulverized through ball mill treatment at 60 rpm for 48 hours . The obtained raw material is hereinafter referred to as separately pulverized raw material.

Examples 1 to 16 and Comparative Examples 1 to 3

0.374 g of the mixed and pulverized raw material (2.00 mmol in terms of $C_6H_{10}O_5$) and an inorganic acid shown in Table 1 were used to provide 40 mL of an aqueous dispersion having a pH adjusted as shown in Table 1. The aqueous dispersion was put in a high-pressure reactor (internal volume: 100 mL, autoclave manufactured by OM LAB-TECH CO., LTD, made of Hastelloy C22), and then, heated from room temperature to a reaction temperature shown in Table 1 at an average rate of temperature increase of 11.3°C/min while being stirred at 600 rpm. After the temperature reached the reaction temperature, the aqueous dispersion was retained at the temperature for a time period shown in Table 1.

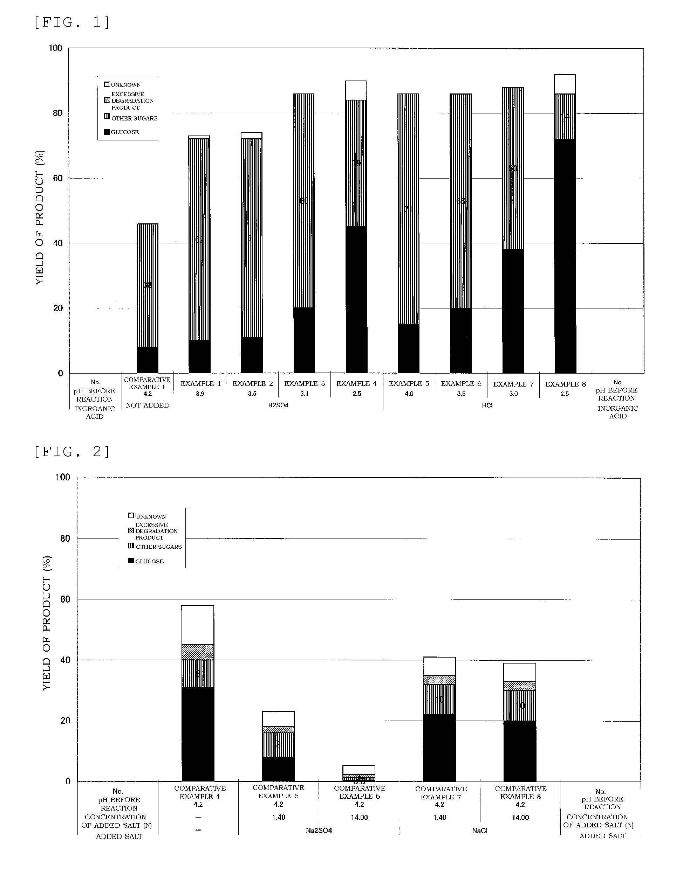

After that, the heating was stopped and the reactor was air-cooled at an average rate of temperature decreaseof16.7°C/min. After the cooling, the reaction liquid was separated with a centrifuge into a liquid and a solid. The products in the liquid phase were quantitatively analyzed for glucose, other sugars, and an excessive degradation product with a high-performance liquid chromatograph manufactured by SHIMADZU CORPORATION (conditions 1 column: Shodex (trademark) SH-1011, mobile phase: water at 0.5mL/min, 50°C, detection: differential refractive index, conditions 2 column: Phenomenex Rezex RPM-Monosaccharide Pb++ (8%), mobile phase: water at 0.6 mL/min, 70°C, detection: differential refractive index). In addition, the solid residues were dried at 110 °C for 24 hours and separated into unreacted cellulose and the carbon catalyst. The rate of conversion of cellulose was determined based on the mass of the unreacted cellulose. The results are shown in Tables 1 and 2 and and FIG. 1. It should be noted that Table 2 shows relative values in the case where values in Comparative Example 1 are defined as 100.

Equations for calculating the yield, rate of conversion of cellulose, and selectivity of glucose are shown below.

$$\text{Yield of product (\%)} = \{(\text{molar number of carbon in component of interest})/(\text{molar number of carbon in added cellulose})\} \times 100$$

$$\text{Rate of conversion of cellulose (\%)} = \{1-(\text{mass of recovered cellulose})/(\text{mass of added cellulose})\} \times 100$$

$$\text{Selectivity of glucose (\%)} = \{(\text{yield of glucose})/(\text{rate of conversion of cellulose})\} \times 100$$

$$\text{Yield of unknown product (\%)} = \text{rate of conversion of cellulose} - \text{total yield of identified components}$$

[Table 1]

| | Condition | | | | Result | | | | | |
| | Added inorganic acid | pH before reaction | Reaction temperature (°C) | Retention time at reaction temperature (min) | Yield of product (%, in terms of carbon) | | | | Rate of conversion of cellulose (%) | Selectivity of glucose (%) |
| | | | | | Glucose | Other sugars a) | Excessive degradation product b) | Unknown | | |
| Example 1 | $H_2SO_4$ | 3.9 | 200 | 0 | 10 | 63 | 1 | 0 | 73 | 14 |
| Example 2 | $H_2SO_4$ | 3.5 | 200 | 0 | 11 | 61 | 2 | 0 | 73 | 15 |
| Example 3 | $H_2SO_4$ | 3.1 | 200 | 0 | 20 | 66 | 2 | 0 | 86 | 23 |
| Example 4 | $H_2SO_4$ | 2.5 | 200 | 0 | 45 | 39 | 3 | 2 | 89 | 51 |
| Example 5 | HCl | 4.0 | 200 | 0 | 14 | 70 | 1 | 0 | 83 | 17 |
| Example 6 | HCl | 3.5 | 200 | 0 | 20 | 66 | 1 | 0 | 85 | 24 |
| Example 7 | HCl | 3.0 | 200 | 0 | 38 | 50 | 2 | 0 | 88 | 43 |
| Example 8 | HCl | 2.5 | 200 | 0 | 72 | 14 | 4 | 3 | 93 | 77 |
| Example 9 | HCl | 2.5 | 200 | 2 | 85 | 6 | 6 | 1 | 98 | 87 |
| Example 10 | HCl | 2.5 | 190 | 5 | 85 | 8 | 5 | 0 | 97 | 87 |
| Example 11 | HCl | 2.5 | 190 | 7 | 87 | 5 | 6 | 1 | 98 | 88 |
| Example 12 | HCl | 2.5 | 180 | 10 | 74 | 19 | 3 | 0 | 96 | 77 |
| Example 13 | HCl | 2.5 | 180 | 20 | 88 | 6 | 5 | 0 | 99 | 89 |
| Example 14 | HCl | 2.5 | 180 | 25 | 88 | 4 | 5 | 0 | 97 | 90 |
| Example 15 | HCl | 2.5 | 170 | 60 | 87 | 6 | 5 | 0 | 97 | 89 |
| Example 16 | HCl | 2.5 | 170 | 75 | 87 | 5 | 6 | 0 | 97 | 89 |
| Comparative Example 1 | Not added | 4.2 | 200 | 0 | 8 | 38 | 1 | 0 | 47 | 17 |
| Comparative Example 2 | Not added | 4.2 | 200 | 2 | 20 | 73 | 2 | 0 | 94 | 21 |

(continued)

| | Condition | | | | Result | | | | | |
|---|---|---|---|---|---|---|---|---|---|---|
| | Added inorganic acid | pH before reaction | Reaction temperature (°C) | Retention time at reaction temperature (min) | Yield of product (%, in terms of carbon) | | | | Rate of conversion of cellulose (%) | Selectivity of glucose (%) |
| | | | | | Glucose | Other sugars a) | Excessive degradation product b) | Unknown | | |
| Comparative Example 3 | Not added | 4.2 | 180 | 20 | 20 | 71 | 2 | 0 | 93 | 22 |
| a) Total of cellobiose, cellotriose, cellotetraose, mannose, and fructose b) Total of levoglucosan, 5-hydroxymethylfurfural, and furfural | | | | | | | | | | |

[Table 2]

|  | Yield of glucose | Rate of conversion of cellulose | Selectivity of glucose |
|---|---|---|---|
| Example 1 | 130 | 160 | 80 |
| Example 2 | 140 | 160 | 89 |
| Example 3 | 250 | 180 | 140 |
| Example 4 | 560 | 190 | 300 |
| Example 5 | 180 | 180 | 100 |
| Example 6 | 250 | 180 | 140 |
| Example 7 | 480 | 190 | 250 |
| Example 8 | 900 | 200 | 460 |
| Example 9 | 1,100 | 210 | 510 |
| Example 10 | 1,100 | 210 | 510 |
| Example 11 | 1,100 | 210 | 520 |
| Example 12 | 920 | 210 | 450 |
| Example 13 | 1,100 | 210 | 520 |
| Example 14 | 1,100 | 210 | 530 |
| Example 15 | 1,100 | 210 | 520 |
| Example 16 | 1,100 | 210 | 530 |
| Comparative Example 1 | 100 | 100 | 100 |

As compared to Comparative Example 1 not using an inorganic acid, each of Examples 1 to 8 using an inorganic acid has an improved rate of conversion of cellulose and an improved yield of glucose. In Examples 1 to 8, the yield of glucose becomes higher as the pH becomes lower, irrespective of the kind of the acid. Of Examples 1 to 8, the conditions under which the best result was obtained were conditions for Example 8 in which the pH was adjusted to 2.5 through addition of hydrochloric acid. In Example 8, the rate of conversion of cellulose was 93%, the yield of glucose was 72%, and the selectivity of glucose was 77%. When the results of Example 8 are expressed as relative ratios to those of Comparative Example 1 not using an inorganic acid, the rate of conversion of cellulose corresponds to 200%, the yield of glucose corresponds to 900%, and the selectivity of glucose corresponds to 460%. This reveals that all the values are significantly improved.

Comparison of the kind of the acid revealed that hydrochloric acid provided more excellent results than sulfuric acid. In particular, significant differences were found in the yield of glucose.

In addition, it is found that, in Example 9, which is the same as Example 8 achieving good results except that the heating time is longer than that in Example 8, the rate of conversion of cellulose, yield of glucose, and selectivity of glucose are further improved.

Further, a reaction was conducted by changing the reaction temperature and the retention time at the reaction temperature under the same conditions as those in Example 8 in which the pH before the reaction was adjusted to 2.5 with hydrochloric acid. In each of the cases of 190°C for 5 minutes (Example 10), 190°C for 7 minutes (Example 11), 180°C for 10 minutes (Example 12), 180°C for 20 minutes (Example 13), 180°C for 25 minutes (Example 14), 170°C for 60 minutes (Example 15), and 170°C for 75 minutes (Example 16), the rate of conversion of cellulose, yield of glucose, and selectivity of glucose were further improved as compared to those in Example 8. It was found that results of the highest level superior to those of Example 8 were obtained by setting a longer retention time even when the reaction temperature was lower than 200°C. The highest values for the rate of yield of glucose, conversion of cellulose, and selectivity of glucose were 88% in Example 13, 99% in Example 13, and 90% in Example 14, respectively.

The fact that Example 12 had a result of 74% lower than the result of the highest level reveals that the conditions under which the results of the highest level are obtained fall within a thermal history range of just enough and appropriate heating. This is because glucose is an intermediate product in hydrolysis of cellulose that is a sequential reaction, and hence, at the same reaction temperature, an excessively short retention time causes poor degradation and an excessively long retention time causes excessive degradation, resulting in a decrease in the yield of glucose.

As a thermal history parameter showing conditions of a reaction temperature and a retention time, Table 3 shows data on a product of temperature and time at a liquid temperature of 160°C or higher (=(treatment temperature-160°C)×time) from temperature increase to temperature decrease for Examples 8 to 16 exhibiting good results. Herein, 160°C, which is a temperature close to the upper limit temperature at which cellulose is not degraded at a retention time of 0 minutes, is used as a standard. It was found that the products of temperature and time in Examples 8 to 16 fell within a range of from 200 to 800°C·min.

An equation for calculating the product of temperature and time at a liquid temperature of 160°C or higher is shown below.

$$
\text{Product of temperature and time at a liquid temperature of } 160°C
$$
$$
\text{or higher} = \{(\text{treatment temperature}-160°C)/\text{rate of temperature}
$$
$$
\text{increase } (°C/min) \times (\text{treatment temperature}-160°C)/2\}
$$
$$
+\{(\text{treatment temperature}-160°C) \times \text{retention time at reaction}
$$
$$
\text{temperature (min)}\}
$$
$$
+\{(\text{treatment temperature}-160°C)/\text{rate of temperature decrease}
$$
$$
(°C/min) \times (\text{treatment temperature}-160°C)/2\}
$$

[Table 3]

|  | Condition | | | |
|---|---|---|---|---|
|  | Reaction temperature (°C) | Retention time at reaction temperature (min) | Product of temperature and time | Yield of glucose (%) |
| Example 8 | 200 | 0 | 240 | 72 |
| Example 9 | 200 | 2 | 320 | 85 |
| Example 10 | 190 | 5 | 280 | 85 |
| Example 11 | 190 | 7 | 340 | 87 |
| Example 12 | 180 | 10 | 260 | 74 |
| Example 13 | 180 | 20 | 460 | 88 |
| Example 14 | 180 | 25 | 560 | 88 |
| Example 15 | 170 | 60 | 620 | 87 |
| Example 16 | 170 | 75 | 770 | 87 |

The results of Comparative Examples 2 and 3, in which the conditions of Examples 9 and 13 exhibiting results of the highest level were adopted except that hydrochloric acid was not added, were as follows: in each of Comparative Examples 2 and 3, the rate of conversion was over 90%, which was slightly lower than those of Examples; the yield and selectivity of glucose were both about 20%, which were both at a level of one-quarter or less of those of Examples; and the yield of other sugars was about 70%, which was around ten times as high as those of Examples. It was found that the addition of hydrochloric acid has an effect of hydrolyzing most of cellooligosaccharides, which are other sugars, to glucose as a monosaccharide without excessively degrading most of the cellooligosaccharides, thereby improving the yield and selectivity

Comparative Examples 4 to 8

0.324 g of the separately pulverized raw material (2.00 mmol in terms of $C_6H_{10}O_5$) , 0.050 g of the carbon catalyst, and a salt weighed in an amount required for a salt concentration shown in Table 4 (N represents normality) were suspended in 40 mL of water. The resultant was put in a high-pressure reactor (internal volume: 100 mL, autoclave manufactured by Nitto Koatsu Co., made of SUS316), and then, heated from room temperature to a reaction temperature of 240°C for about 15 minutes while being stirred at 600 rpm. On reaching the reaction temperature, the heating was stopped and the reactor was put in a water tank to be cooled. After the cooling, the reaction liquid was separated with a centrifuge into a liquid and a solid. The products in the liquid phase were quantitatively analyzed with a high-performance liquid chromatograph (apparatus: Shodex high-performance liquid chromatography manufactured by Showa Denko K.K., column: Shodex (trademark) KS801, mobile phase: water at 0.6mL/min, 75°C, detection: differential refractive index). In addition, solid residues were washed with water and dried at 110°C for 24 hours. Then, the rate of conversion of cellulose was determined based on a mass of unreacted cellulose. The results are shown in Tables 4 and 5 and FIG. 2.

[Table 4]

| | Condition | | | Result | | | | | |
|---|---|---|---|---|---|---|---|---|---|
| | Added inorganic acid | Concentration (mN) | pH before reaction | Yield of product (%, in terms of carbon) | | | | Rate of conversion of cellulose (%) | Selectivity of glucose (%) |
| | | | | Glucose | Other sugars a) | Excessive degradation product b) | Unknown | | |
| Comparative Example 4 | Not added | - | 4.2 | 31 | 9 | 5 | 13 | 58 | 53 |
| Comparative Example 5 | $Na_2SO_4$ | 1.40 | 4.1 | 8 | 8 | 2 | 5 | 23 | 35 |
| Comparative Example 6 | $Na_2SO_4$ | 14.00 | 4.3 | 0.3 | 1 | 1 | 3 | 5 | 6 |
| Comparative Example 7 | NaCl | 1.40 | 4.2 | 22 | 10 | 3 | 6 | 41 | 54 |
| Comparative Example 8 | NaCl | 14.00 | 4.2 | 20 | 10 | 3 | 6 | 39 | 51 |
| a) Total of cellobiose, cellotriose, cellotetraose, mannose, and<br>b) Total of levoglucosan and 5-hydroxymethylfurfural | | | | | | | | | |

[Table 5]

| | Relative ratio (%) to Comparative Example 4 | | |
|---|---|---|---|
| | Yield of glucose | Rate of conversion of cellulose | Selectivity of glucose |
| Comparative Example 4 | 100 | 100 | 100 |
| Comparative Example 5 | 26 | 40 | 65 |
| Comparative Example 6 | 1 | 9 | 11 |
| Comparative Example 7 | 71 | 71 | 100 |
| Comparative Example 8 | 65 | 67 | 96 |

It was confirmed that, in each of the cases where $Na_2SO_4$ or NaCl was added, the rate of conversion of cellulose and yield of glucose were reduced and the reaction was inhibited as compared to Comparative Example 4 adopting a condition of adding no inorganic salt.

This does not mean that an anion such as $SO_4^{2-}$ and Cl- suffices, and indicates that the pH is important.

Industrial Applicability

In a hydrolysis reaction of a plant biomass using a solid catalyst, the present invention can improve the yield of glucose and selectivity of glucose by a simple method including adjusting a pH through addition of an inorganic acid. The present invention is extremely useful for effective utilization of biomass resources.

**Claims**

1. A method of hydrolyzing a plant biomass, including a step of heating a mixture containing a plant biomass, a solid catalyst for catalyzing hydrolysis of the biomass, an inorganic acid, and water.

2. The method of hydrolyzing a plant biomass according to claim 1, in which the mixture containing a plant biomass, a solid catalyst, an inorganic acid, and water has a pH of from 1.0 to 4.0.

3. The method of hydrolyzing a plant biomass according to claim 2, in which the mixture containing a plant biomass, a solid catalyst, an inorganic acid, and water has a pH of from 2.0 to 3.0.

4. The method of hydrolyzing a plant biomass according to any one of claims 1 to 3, in which the inorganic acid includes at least one kind selected from hydrochloric acid, sulfuric acid, nitric acid, phosphoric acid, and boric acid.

5. The method of hydrolyzing a plant biomass according to any one of claims 1 to 3, in which the inorganic acid is hydrochloric acid.

6. The method of hydrolyzing a plant biomass according to any one of claims 1 to 5, in which the heating is performed at a maximum heating (reaction) temperature of from 170°C to 200°C and a retention time at the temperature is from 0 to 120 minutes.

7. The method of hydrolyzing a plant biomass according to any one of claims 1 to 6, in which the heating is performed so that, in a graph with a vertical axis representing a reaction temperature and a horizontal axis representing time, a product of temperature and time for a portion above 160°C ((treatment temperature-160°C)×time) is from 200 to 800°C·min.

8. The method of hydrolyzing a plant biomass according to any one of claims 1 to 7, in which the solid catalyst includes a carbon material.

9. The method of hydrolyzing a plant biomass according to claim 8, in which the carbon material includes alkali-activated carbon, steam-activated carbon, or mesoporous carbon.

**10.** The method of hydrolyzing a plant biomass according to any one of claims 1 to 9, in which the plant biomass includes cellulose.

**11.** A method of producing glucose, comprising performing the method of hydrolyzing a plant biomass according to any one of claims 1 to 10.

EP 2 871 246 A1

[FIG. 1]

[FIG. 2]

## INTERNATIONAL SEARCH REPORT

International application No.

PCT/JP2013/068277

### A. CLASSIFICATION OF SUBJECT MATTER

*C13K1/02*(2006.01)i, *B01J21/18*(2006.01)i, *B09B3/00*(2006.01)i

According to International Patent Classification (IPC) or to both national classification and IPC

### B. FIELDS SEARCHED

Minimum documentation searched (classification system followed by classification symbols)

C13K1/02, B01J21/18, B09B3/00

Documentation searched other than minimum documentation to the extent that such documents are included in the fields searched

| | | |
|---|---|---|
| Jitsuyo Shinan Koho | 1922-1996 | Jitsuyo Shinan Toroku Koho 1996-2013 |
| Kokai Jitsuyo Shinan Koho | 1971-2013 | Toroku Jitsuyo Shinan Koho 1994-2013 |

Electronic data base consulted during the international search (name of data base and, where practicable, search terms used)

JSTPlus/JMEDPlus/JST7580(JDreamIII), WPI

### C. DOCUMENTS CONSIDERED TO BE RELEVANT

| Category* | Citation of document, with indication, where appropriate, of the relevant passages | Relevant to claim No. |
|---|---|---|
| X | JP 2006-129735 A  (Hiroshima University), 25 May 2006 (25.05.2006), claims; columns 0042, 0044 (Family: none) | 1-11 |
| X | JP 2011-526484 A  (Sud-Chemie AG.), 13 October 2011 (13.10.2011), claims 1, 8; columns 0038, 0040; examples 3, 4, 6 & WO 2010/009958 A1    & US 2011/0152514 A1 & EP 2294230 A1          & DE 102008030892 A1 | 1-11 |
| A | WO 2011/087131 A1  (IHI Corp.), 21 July 2011 (21.07.2011), claims; columns 0032 to 0034 & US 2012/0279495 A1    & CN 102892905 A & JP 2011-142892 A | 1-11 |

☒ Further documents are listed in the continuation of Box C.      ☐ See patent family annex.

| | |
|---|---|
| *    Special categories of cited documents: <br> "A"   document defining the general state of the art which is not considered    to be of particular relevance <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| | |
|---|---|
| Date of the actual completion of the international search <br>     24 July, 2013 (24.07.13) | Date of mailing of the international search report <br>     06 August, 2013 (06.08.13) |
| Name and mailing address of the ISA/ <br>     Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2013/068277

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2011-206044 A  (Sekisui Chemical Co., Ltd.), 20 October 2011 (20.10.2011), claims; tables 7, 8 (Family: none) | 1-11 |
| A | JP 2009-201405 A  (National University Corporation Kochi University), 10 September 2009 (10.09.2009), entire text (Family: none) | 1-11 |
| A | JP 2012-110873 A  (Tanaka Kikinzoku Kogyo Kabushiki Kaisha), 14 June 2012 (14.06.2012), column 0031 (Family: none) | 9 |

Form PCT/ISA/210 (continuation of second sheet) (July 2009)

**EP 2 871 246 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2008297229 A
- JP 2010098994 A
- JP 2009201405 A
- JP 2008271787 A
- US 8382905 B2
- JP 2011206044 A